# EUROPEAN PATENT APPLICATION

(11) **EP 3 831 444 A1**
(43) Date of publication of application: **09.06.2021**
(21) Application number: 20155422.7
(22) Date of filing: 04.02.2020
(51) Int. Cl.: A61N 1/375

(54) **HEADER ASSEMBLY AND IMPLANTABLE MEDICAL DEVICE**

(30) Priority: 06.12.2019 US 201962944381 P
(71) Applicant: BIOTRONIK SE & Co. KG, 12359 Berlin (DE)
(72) Inventor: HOLMES, Aaron, Happy Valley, OR 97086 (US); BRUNNER, Bjoern, Portland, OR 97219 (US)
(74) Representative: Heinz, Benjamin

(57) **Abstract**

A header assembly (20, 51) for an implantable medical device is disclosed, wherein the header assembly comprises a lead aperture (21) extending in a longitudinal direction (22), wherein the lead aperture (21) comprises a first contact element (31) and a second contact element (32, 52) accommodated in a pre-defined distance from each other in longitudinal direction, wherein the first contact element is electrically connectable to a first terminal of a power source of the implantable medical device and the second contact element is electrically connectable to a second terminal of the power source, wherein the first contact element forms a first contact area and the second contact element forms a second contact area (62), wherein an electrically conducting pin (44) is located within the lead aperture, wherein the pin is movable along the longitudinal direction of the lead aperture from an initial position to a second position such that in the second position the pin provides an electrical connection of the first contact area and the second contact area and in the initial position said electrical connection is open. Further, an implantable medical device with such header assembly is described. The header assembly and the medical device provide a robust, less costly and less complicated solution for enhancement of longevity of implantable medical devices.

## Description

Implantable medical devices for providing electrical stimulation to body tissues, for monitoring physiologic conditions, and for providing alternative treatments to drugs are well-known in the art. Exemplary implantable medical devices include implantable monitors, cardio defibrillators, pace makers, and programmable neuro-stimulator pulse generators. The medical devices typically incorporate a power source connected with an electronic circuit having a circuit board forming an electronic module accommodated within a hermetically sealed device housing. Connected to the sealed housing often a header assembly is provided which includes electrical contact elements that are electrically coupled to the electronic circuit and/or to the power source located inside the housing via a feedthrough component. The header assembly provides a connector means for electrically communicating via an external medical lead cable.

Ensuring maximum battery life prior to implantation is critical to longevity of implantable medical devices. In order to cope with this problem nowadays often costly control of the supply chain is implemented with the goal to minimize the time between battery connection and implantation in patients.

Document US 5,350,407 discloses an implantable stimulator including an aperture or socket adapted to accept either a stimulation lead or an activation pin, and a circuit that is adapted to detect removal of an inserted activation pin. Upon completion of manufacture, and before being placed on a shelf to await implantation in a patient, the activating pin is inserted into the stimulator socket. Then, the external communicating device sends a deactivating command to the implantable stimulator. The stimulator responds by generally disabling power to stimulator circuits while maintaining power to the oscillator, and generally disabling operations of the stimulator while maintaining the operations of the stimulator associated with communication operations. Later, the stimulator may be activated by removing the activation pin from the stimulator aperture. In response to the removal of the activating pin, the stimulator enables the previously disabled operations and enables power to the previously unpowered stimulator circuits. This is, however, a not very safe method of deactivation or activation of a medical device as the pin can be removed by accident. Additionally, the detection of removal of the pin seems to be too complicated and cost intensive.

Accordingly, there is a desire for a robust, less costly and less complicated solution for enhancement of longevity of implantable medical devices. Further, the mechanism of the implantable device must be safe to handle.

The above task is solved by the following embodiments of a header assembly for an implantable medical device and a respective medical device.

In one embodiment the header assembly comprises a lead aperture (socket) extending in a longitudinal direction, wherein the lead aperture comprises a first contact element and a second contact element accommodated in a pre-defined distance from each other, wherein the first contact element is electrically connectable to a first terminal (e.g. an anode) of a power source, e.g. a battery, of the implantable medical device and the second contact element is electrically connectable to a second terminal (e.g. a cathode) of the power source, wherein the first contact element forms a first contact area and the second contact element forms a second contact area. Further, an electrically conducting pin is located within the lead aperture, wherein the pin is movable along the longitudinal direction of the lead aperture from an initial position to a second position such that in the second position the pin provides an electrical connection of the first contact area and the second contact area and in the initial position said electrical connection is open. The power source of the implantable medical device is electrically connected to an electronic module located within a hermetically sealed housing of the device forming an electronic circuit. In a shelf state the first contact area and the second contact area are not electrically connected, i.e. in the initial position of the pin of the header assembly and the electrical circuit is open. No power of the power source is consumed. In the second position, i.e. in the operational state, as soon as the electrical connection of the first contact area and the second contact area is established, the electrical circuit comprising the electronic module of the implantable device is closed, thereby ensuring pre-defined operation of the implantable device.

At the end of manufacturing the medical device is provided with the header assembly, wherein the pin of the header assembly is in the initial position, i.e. in the shelf state, in which there is no electrical connection of the first contact area and the second contact area so that the electrical circuit is open. If the implantation of the medical device is envisaged, the pin of the header assembly is moved along the longitudinal direction of the aperture by a tool, e.g. an Allen wrench, so that the electrical connection of the first contact area and the second contact area is established thereby transferring the medical device from the shelf state into the operational state as well as closing the electrical circuit and activating the medical device. In the shelf state and in the operational state the pin is fixed in each position within the lead aperture. Further, the pin is guided (by a thread or another guiding element) from the initial position to the operational state. Accordingly, the operational state cannot be taken by accident so that the above solution is safe to handle.

The above header assembly and respective implantable medical device provide a robust mechanism accessible through the device header, for example along the longitudinal direction of the aperture, that minimizes the drain on the power source during shelf life. It eliminates wasted device due to shelf-life expiration of the devices and reduces costs for shipping, storage, and maintaining of such implantable medical devices after assembly stages.

In one embodiment the first contact area and/or the second contact area each form a section of the aperture wall. For example, the first contact element and/or the second contact element is a conductive block with a through-hole, wherein the through-hole forms a section of the lead aperture. The conductive block may have the form of a cuboid, cube, or similar, and may comprise rounded corners and edges or may at least partially be formed as a section of a sphere or may be formed fully as a sphere.

In one embodiment the pin may comprise an outer thread, wherein the pin is movable along the longitudinal direction by screwing along a thread located at the wall of the lead aperture. Alternatively, the pin may comprise one of a guiding recess or guiding projection, wherein the wall of the lead aperture comprises the other one of the guiding recess or the guiding projection. Thereby, a precise movement of the pin and a safe handling is ensured.

Additionally, an easy adjustment of the pin within the lead aperture is provided if one end face of the pin comprises a profile allowing an external tool (e.g. an Allen wrench or similar) to screw the pin, e.g. a hexagonal recess or a slit or a respective projection. When inserted in the lead aperture the end face of the pin points into the direction of an opening of the lead aperture at the longitudinal direction. The external tool comprises a profile which is a counterpart the profile at the pin.

In one embodiment the first contact area and/or the second contact area is formed by a cantilevered contact spring. This embodiment provides an exact positioning of the pin and a good contact of the contact area of the first and the second contact element. In one embodiment the contact element is a pin-like element inserted into a respective pocket of the header assembly formed an end section of the lead aperture and the cantilevered contact spring projects from the pin-like element into the lead aperture. The pin-like contact element comprises lateral projecting wings for fixation in the pocket after insertion.

In one embodiment, the header assembly comprises a molded insulator body, in which the lead aperture is formed. This header assembly can be manufactured at low costs.

According to embodiments of the invention, the material for the molded header can comprise epoxy, two part epoxy or silicone. The material for the contact element can comprise at least one of a titanium alloy, platinum iridium or stainless steel. The material for the pin may comprise at least one of titanium, titanium alloys, platinum iridium, or stainless steel.

In one embodiment an implantable medical device comprising a header assembly and a power source is provided, wherein the first contact element and its contact area is electrically connected to a first terminal of the power source of the implantable medical device and the second contact element and its contact area is electrically connected to a second terminal of the power source.

In one embodiment the medical device comprises a controlling unit, wherein the controlling unit is adapted to start a self-test sequence as soon as the pin has established an electrical contact between the first contact area of the first contact element and the second contact area of the second contact element, i.e. in the second position of the pin or the operational state of the header assembly.

In one embodiment, the electrical connection of the first contact element and the second contact element (and their respective contact areas) to the power source is provided by at least one feedthrough assembly. The feedthrough assembly provides a safe and robust electrical connection.

A full and enabling disclosure of the present invention, including the best mode thereof, directed to one of ordinary skill in the art is set forth in the following specification. Thereby, further features and advantages are presented that are part of the present invention independently of the features mentioned in the claims.

The specification refers to the accompanying figures showing schematically and partly transparent
- Fig. 1: a first embodiment of a header assembly in a partial perspective side view,
- Fig. 2: the embodiment of Fig. 1 in a partial front view,
- Fig. 3: the embodiment of Fig. 1 in a side view with a tool,
- Fig. 4: a part of the header assembly of Fig. 1 in a perspective side view, during a first manufacturing step,
- Fig. 5: the part of Fig. 4 in a front view,
- Fig. 6: the part of Fig. 4 during a second manufacturing step in a perspective side view,
- Fig. 7: the part of Fig. 6 in a front view,
- Fig. 8: the part of Fig. 4 during a third manufacturing step in a perspective side view and in the initial position of the pin,
- Fig. 9: the part of Fig. 4 in a side view, after activation of the medical device,
- Fig. 10: a second embodiment of a header assembly in a partial perspective side view,
- Fig. 11: the embodiment of Fig. 10 in a partial front view,
- Fig. 12: the embodiment of Fig. 10 in a side view with a tool,
- Fig. 13: a part of the header assembly of Fig. 10 in a side view in the initial position of the pin,
- Fig. 14: the part of Fig. 13 in a side view, after activation of the medical device,
- Fig. 15: the part of Fig. 13 in a perspective side view,
- Fig. 16: the part of Fig. 13 in a perspective side view, during a first manufacturing step,
- Fig. 17: the part of Fig. 16 in a front view,
- Fig. 18: the part of Fig. 13 during a second manufacturing step in a perspective side view,
- Fig. 19: the part of Fig. 18 in a top view, and
- Fig. 20: the part of Fig. 13 during a third manufacturing step in a perspective side view, and
- Fig. 21: side view of an exemplary implantable medical device comprising a header portion and a housing.

The medical device according to the invention, for example an implantable pace maker, comprises a housing (not shown), a power source (not shown), e.g. a battery, and an electronic circuit (not shown) provided on a circuit board (e.g. printed circuit board, PCB) forming an electronic module. The electronic circuit is electrically connected to the power source and is located together with the power source in the housing which is hermetically sealed against the body fluid of a patient.

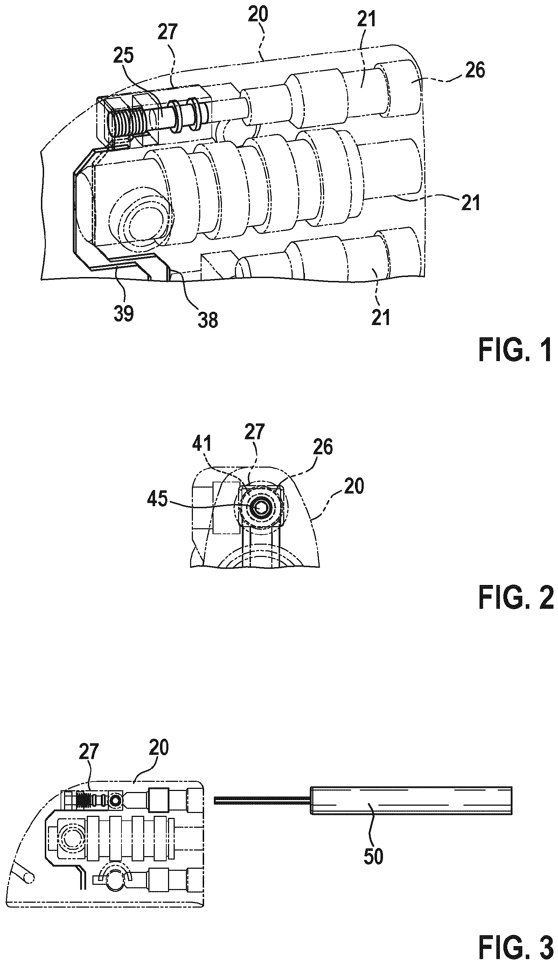

The medical device 5 further comprises a header assembly 20, the first embodiment of at least a part of it is shown partly transparent in Fig. 1 and in Fig. 2 to 9. The header assembly 20 includes three lead apertures 21 for electrical connection with lead cables. It is possible to have a different number of lead apertures - this is dependent on the respective medical device. Further, the header assembly provides at least one feedthrough assembly (not shown) comprising electrical connections to respective terminals of the electronic module provided on the circuit board and/or to respective terminals (anode and cathode) of the power source.

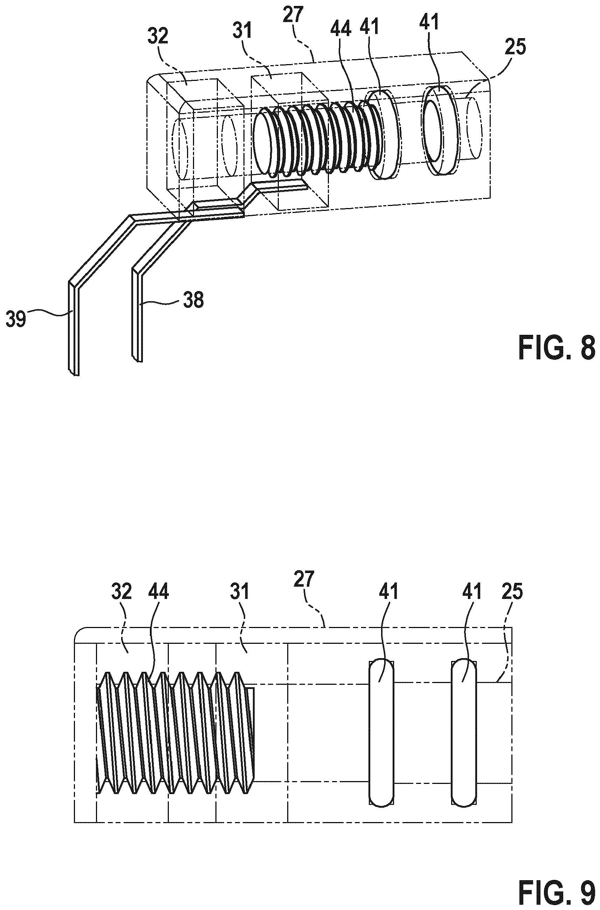

All elements of the header assembly 20 are encapsulated in a molded biocompatible, insulating material, for example two part epoxy
- The most upper lead aperture (socket) 21 extends in longitudinal direction 22 and comprises an end section 25 situated opposite to the opening 26 for insertion of the lead cable. This end section 25 is formed by a molded block 27 (see Fig. 8 for details) constituting a part of the header assembly 20. The mold material of the molded block 27 and the whole header assembly 20 are preferably identical. The (inner) wall of the end section 25 has a thread and is formed at least partially by a first contact element 31 and a second contact element 32 accommodated in a pre-defined distance from each other in longitudinal direction 22. Both contact elements 31, 32 are box-shaped electrically conductive blocks, each with a respective through-hole 33, 34. Each through-hole 33, 34 forms a section of the lead aperture 21. The wall of the through-hole 33 of the first contact element 31 forms a first contact area and the wall of the through-hole 34 of the second contact element 32 forms a second contact area. At its lower surface, the box-shaped first contact element 31 is electrically connected to a first lead 38, which in turn is electrically connected to the at least one feedthrough assembly. Similarly, at its lower surface, the box-shaped second contact element 32 is electrically connected to a second lead 39, which in turn is electrically connected to the at least one feedthrough assembly. The first contact element 31 and the second contact element 32 comprise electrically conducting material, for example titanium alloys, platinum iridium, or stainless steel. The electrically conducting material is located at least at the wall of the through-hole 33, 34 of the respective contact element 31, 32 and at the contact surface for the respective lead 38, 39.

Further, the end section 25 of the lead aperture 21 comprises two O-rings 41 located in a pre-defined distance from each other, wherein the inner surface of each O-ring 41 forms a section of the lead aperture 21. The O-rings 41 are located closer to the opening 26 for insertion of the respective lead cable than the first contact element 31 and the second contact element in longitudinal direction 22.

Further, the lead aperture 21 comprises a cylindrical pin 44 having a thread at its outer shell surface. The pin 44 comprises electrical conducting material, for example at its outer surface. In an initial position or a shelf state shown in Fig. 1, 3, 6 and 8, the pin 44 is electrically connected to the first contact element 31 by the meshing thread at the outer surface of the pin 44 and the inner surface of the through-hole 33 of the first contact element 31. At the end face pointing in the direction of the opening 26 of the lead aperture 21 the pin 44 comprises a profile, e.g. a hexagonal recess 45, allowing an external tool having the counterpart of this profile, e.g. an Allen wrench 50, to screw the pin 44 such that it moves (i.e. screws) in longitudinal direction 22 along the lead aperture 21. In the initial state an electronic circuit (not shown) comprising the power source, the at least one feedthrough-assembly, the first lead 38, the second lead 39, at least a part of the electronic module is open because there is no electrical connection between the first contact element 31 and the second contact element 32 or their respective contact areas. Accordingly, no power of the power source is consumed.

In case the implantation of the medical device is envisaged the pin 44 is screwed along the longitudinal direction 22 so far that it is screwed into the through-hole 34 of the second contact element 32 (see Fig. 9). By meshing the outer thread of the pin 44 and the thread of the second contact element 32 an electrical connection of the second contact element 32 and the pin 44 is established while there is still an electrical connection of the first contact element 31 and the pin 44. Thereby an electrical connection of the first contact element 31 and the second contact element 32 (or their respective contact areas) is provided. Thereby the medical device is transferred from the shelf state into the operational state in which the electronic circuit comprising the first contact element 31 and the second contact element 32 is closed. Accordingly, the medical device is activated and power of the power source is consumed. After activation the medical device operates as intended. In one embodiment during activation of the medical device, a boot-up self-test sequence is executed once the electrical connection of the first contact element 31 and the second contact element 32 is established. For this, the electronic module comprises a communication unit to provide appropriate communication with an external operator (programmer) to ensure full functionality once the medical device is energized. The above explained inventive solution provides a robust mechanical switch on/off mechanism accessible through the header assembly at the time of implant.

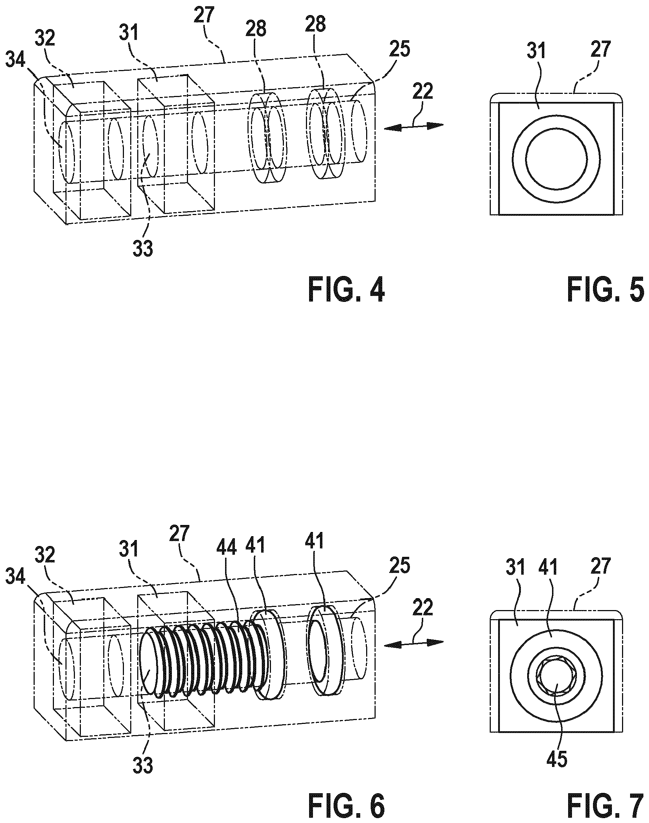

As shown in Fig. 4 to 8, the molded block 27 comprising the end section 25 of the lead aperture 21 is manufactured as follows. As shown in Fig. 4 and 5, in the first step the first contact element 31 and the second contact element 32 are placed with a pre-defined distance from each other and are molded over by a biocompatible, insulating and (see above) thereby forming the molded block 27. A center hole is machined through the molded block and contacts 31 and 32 in the direction shown 22 then tapped. O-ring grooves 28 are then machined into molded block 27.

Then, the pin 44 is inserted into the end section 25 of the lead aperture 21 and screwed along its longitudinal direction 22 so that it is electrically connected with the first contact element 31 by the meshing thread. For example, the pin 44 is screwed back a location fixed by the O-ring 41 which is located close to the first contact element 31 (see Fig. 6 and 8). Accordingly, the contact area of the first contact element 31 and the contact area pin 44 are electrically connected but not the second contact element 32 (or its contact area). Thereby the shelf mode is established. The above mentioned electronic circuit of the medical device comprising the power source is open once the header is attached to the remaining elements of the medical device.

Further, the O-rings 41 are placed within their respective recesses. The O-ring 41 located closer to the opening 26 seals on the tip of a lead placed within the lead aperture 21. As indicated above, the second O-ring 41 accommodated closer to the first contact element 31 serves for orientation during screw in of the pin 44 into its initial position.

Afterwards, the first lead 38 and the second lead 39 are welded to a conducting surface of the respective contact element 31, 32 and connected to the respective contact of the at least one feedthrough assembly. Finally, the molded block 27 with the leads 38, 39 are located in-line with lead insertion location and mold header assembly (see Fig. 1) and the header assembly 20 is attached to and electrically connected to the remaining part of the medical device, e.g. using a feedthrough assembly.

For implantation the above explained activation of the medical device is provided.

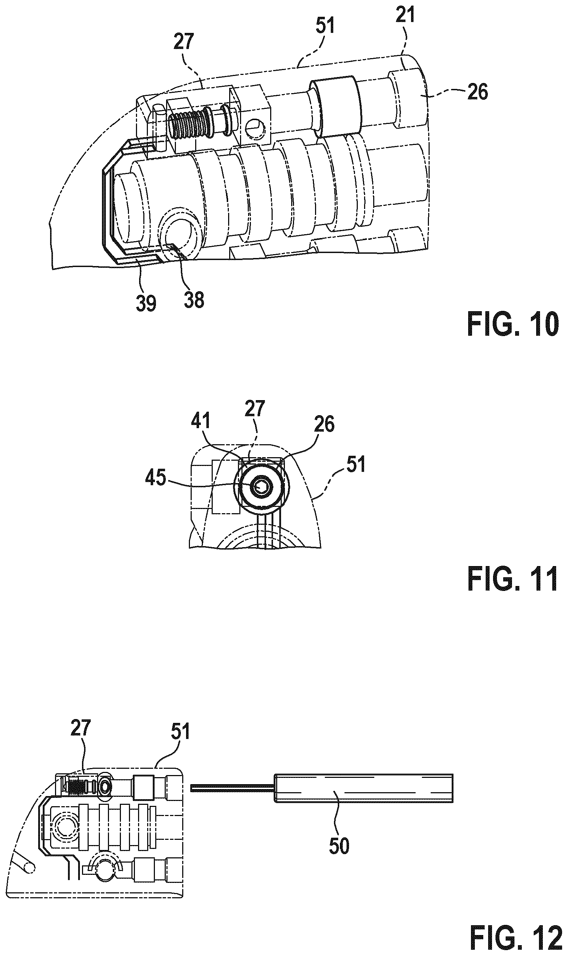

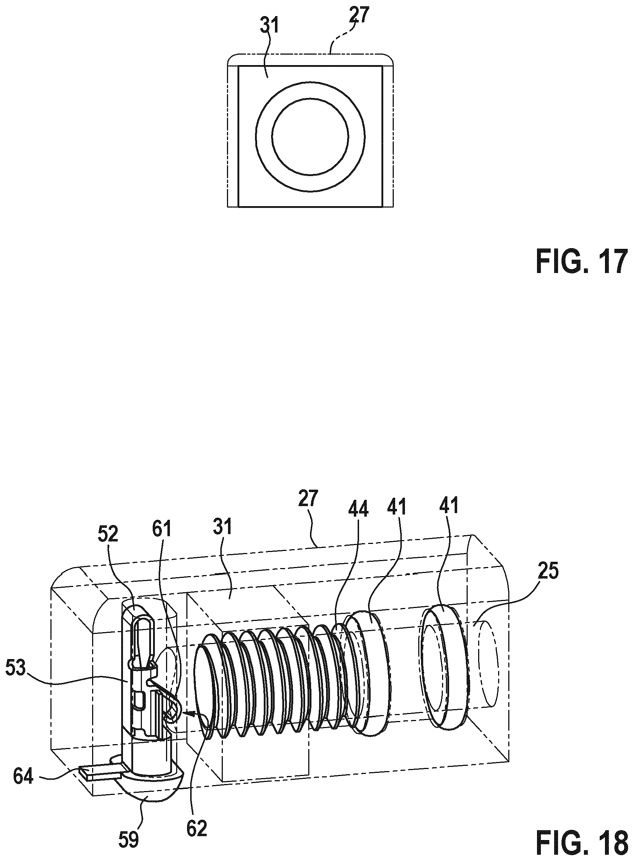

A second embodiment of a header assembly 51 is explained using Fig. 10 to 20. This embodiment comprises a different second contact element 51 compared with the first embodiment. The remaining construction of the header assembly 51 or the medical device and their manufacturing is similar to the first embodiment. Accordingly, the same reference numbers are used for similar elements and it is referred to the above explanations with regard to the first embodiment which also apply to the second embodiment.

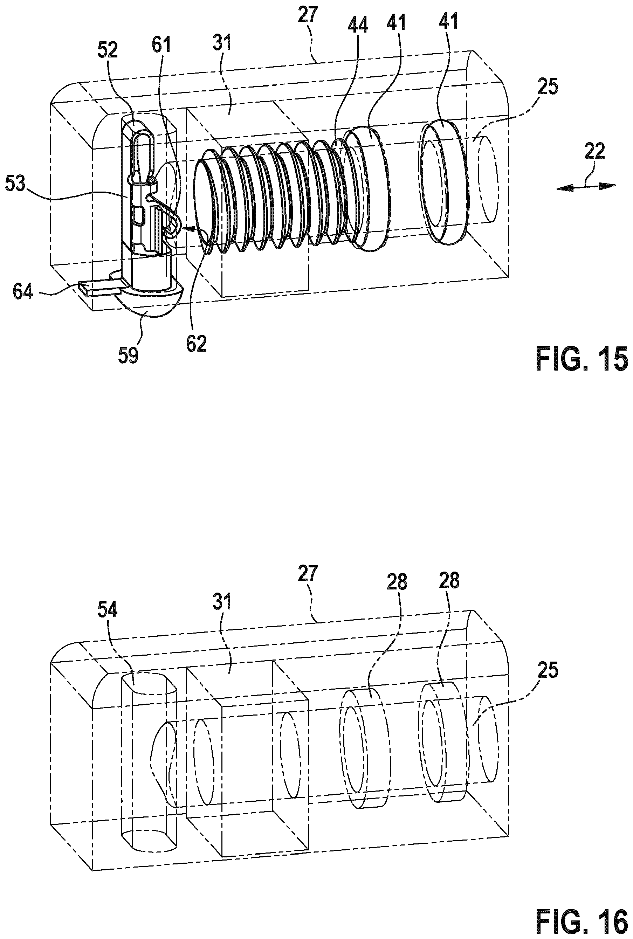

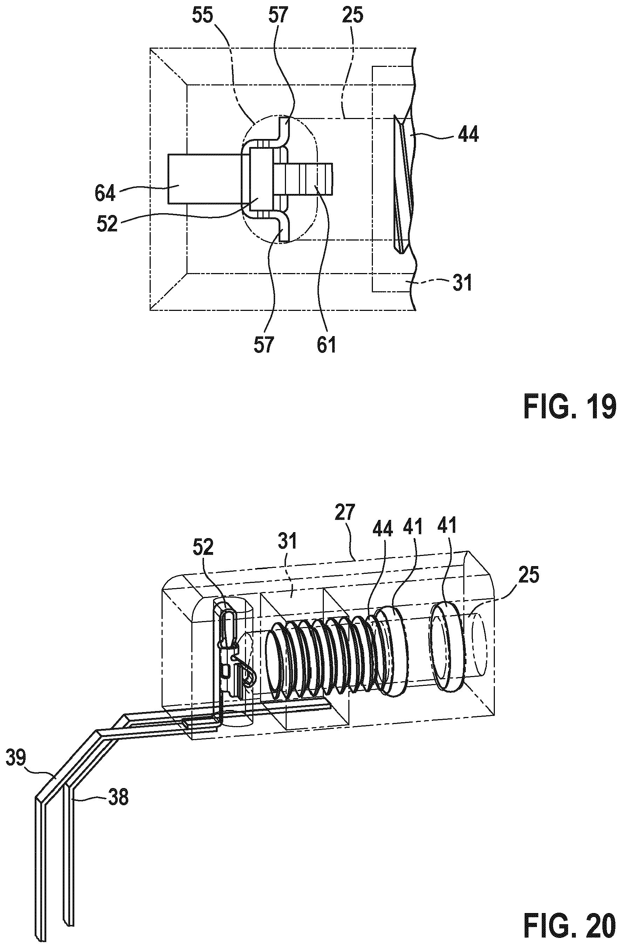

The second contact element 52 of the second embodiment of the header assembly 51 has a pin-like body 53 and is inserted in a respective pocket 54 in the molded block 27 (see Fig. 16) located at the furthest end of the end section 25 of the lead aperture 21 opposite to the opening 25. The pocket 54 is larger in a pre-defined section 55 (see Fig. 19) for secure fixation of lateral spring-like wings 57 of the second contact element 52 after insertion. The second contact element 52 is inserted by means of a mushroom-shaped plug 59 into the pocket 54 for precise location of the second contact element 52 within the pocket 54. The contact element 52 and the plug 59 are inserted into a direction perpendicular to the longitudinal direction 22 up to a stop provided by the plug 59. Afterwards, the plug 59 is removed.

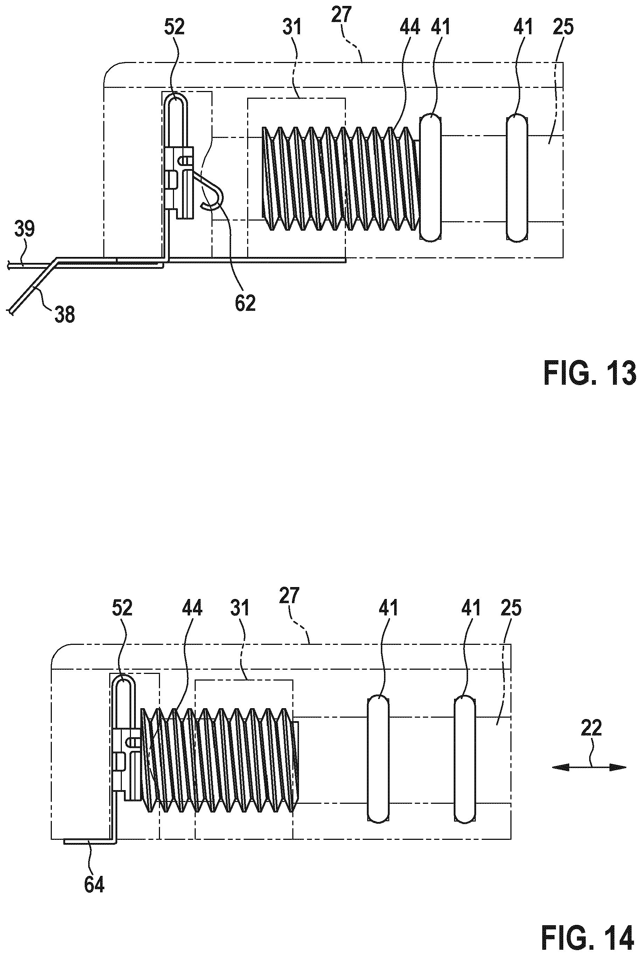

Further, the second contact element 52 comprises a cantilevered contact spring 61 forming a contact area 62 for the pin 44 at a turning section of the spring 61. If the second contact element 52 is fully inserted into the pocket 54, the contact spring 61 with the contact area 62 projects into the end section 25 of the lead aperture 21. For connection with the second lead 39 the second contact element 52 comprises a contact 64 at its lower end. The lead 39 for the electrical connection to the electronic module is welded to the contact 64 thereby forming an electrical connection (see Fig. 13 and 20).

As indicated in Fig. 14, for activation the medical device the pin 44 is screwed along the longitudinal direction 22 as long as the end face of the pin 44 (the end face opposite the end face with the profile for the tool) touches the contact area 62 of the contact spring 61 thereby establishing an electrical connection. The pin 44 may be screwed by the Allen wrench 50 in longitudinal direction as long as the pin 44 is hard stopped when the contact spring 61 strikes the body 53 of the second contact element 52.

In both embodiments, the tool for moving the pin 44 (e.g. Allen wrench 50) may comprise a detector for ensuring correct electrical connection of the pin 44 and the respective contact area of the second contact element 32, 52. For example, a spring compliant Allen wrench may be used with torque setting.

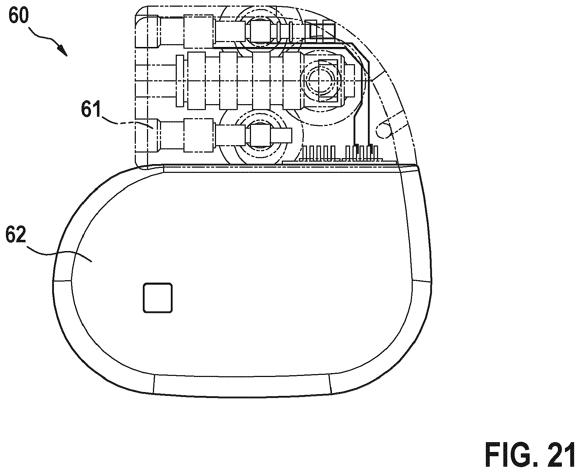

Fig. 21 shows a side view of an exemplary (implantable) medical device 60 comprising a header portion 61 as known in prior art and a housing 62. Housing 62 typically encapsulates sensitive electronic components as e.g. the power source and electronic module(s).

The above described embodiments of a header assembly and the respective implantable medical devices enable changes in the distribution model for implantable devices and eliminate wasted devices due to shelf-life expiration.

### LIST OF REFERENCE SIGNS

- 60: medical device
- 61: header assembly (prior art)
- 62: housing
- 20, 51: header assembly
- 21: lead aperture
- 22: longitudinal direction
- 25: end section of lead aperture 21
- 26: opening
- 27: molded block
- 28: recess
- 31: first contact element
- 32: second contact element
- 33: through-hole
- 34: through-hole
- 38: first lead
- 39: second lead
- 41: O-ring
- 44: pin
- 45: hexagonal recess
- 50: Allen wrench
- 52: second contact element
- 53: body of second contact element 52
- 54: pocket
- 55: section of pocket
- 57: wing of second contact element 52
- 61: contact spring
- 62: contact area

## Claims

1. A header assembly (20, 51) for an implantable medical device, wherein the header assembly comprises a lead aperture (21) extending in a longitudinal direction (22), wherein the lead aperture (21) comprises a first contact element (31) and a second contact element (32, 52) accommodated in a pre-defined distance from each other in longitudinal direction,
wherein the first contact element is electrically connectable to a first terminal of a power source of the implantable medical device and the second contact element is electrically connectable to a second terminal of the power source,
wherein the first contact element forms a first contact area and the second contact element forms a second contact area (62),
wherein an electrically conducting pin (44) is located within the lead aperture, wherein the pin is movable along the longitudinal direction of the lead aperture from an initial position to a second position such that in the second position the pin provides an electrical connection of the first contact area and the second contact area and in the initial position said electrical connection is open.

2. The header assembly of claim 1, wherein the first contact area and/or the second contact area each form a section of the aperture wall.

3. The header assembly of claim 2, wherein the first contact element and/or the second contact element is an electrically conductive block with a through-hole (33, 34), wherein the through-hole forms a section of the lead aperture.

4. The header assembly of any of the previous claims, wherein the pin comprises an outer thread, wherein the pin is movable along the longitudinal direction by screwing along a thread located at the wall of the lead aperture.

5. The header assembly of claim 4, wherein one end face of the pin comprises a profile (45) allowing an external tool (50) to screw the pin.

6. The header assembly of any of the previous claims, wherein the first contact area and/or the second contact area (62) is formed by a cantilevered contact spring (61).

7. The header assembly of claim 6, wherein the contact element (52) is a pin-like element inserted into a respective opening (54) of the header assembly forming an end section of the lead aperture, such that the cantilevered contact spring (61) projects from the pin-like element into the lead aperture.

8. The header assembly of any of the previous claims, wherein the header assembly comprises a molded insulator body, in which the aperture is formed.

9. The header assembly of any of the previous claims, wherein the header is formed of a material comprising at least one of epoxy, two-part epoxy or silicone.

10. An implantable medical device comprising a header assembly of any of the previous claims and a power source, wherein the first contact element is electrically connected to a first terminal of the power source of the implantable medical device and the second contact element is electrically connected to a second terminal of the power source.

11. The implantable medical device of claim 10, wherein the device comprises a controlling unit, wherein the controlling unit is adapted to start a self-test sequence as soon as the pin has established an electrical contact between the first contact area and the second contact area.

12. The implantable medical device of any of the claims 10 to 11, wherein the electrical connection of the first contact element and the second contact element to the power source is provided via a feedthrough component.
